# FASCICULE DE BREVET EUROPEEN

(11) **EP 2 051 720 B1**
(45) Date de publication et mention de la délivrance du brevet: **25.04.2012**
(21) Numéro de dépôt: 07765771.6
(22) Date de dépôt: 03.07.2007
(51) Int. Cl.: A61K 35/56, C12N 5/07

(54) **PROCEDE DE MATURATION OVOCYTAIRE**
OOZYTEN-REIFUNGSMETHODE
OOCYTE MATURATION METHOD

(30) Priorité: 03.07.2006 WO PCT/EP2006/006450
(43) Date de publication de la demande: 29.04.2009
(73) Titulaire: Université Libre de Bruxelles, 1050 Bruxelles (BE); Institut Halieutique et des Sciences Marines de l'Université de Toliara, 601 Toliara (MG); Université de Mons-Hainaut, 7000 Mons (BE)
(72) Inventeur: JANGOUX, Michel, 1050 Bruxelles (BE); EECKHAUT, Igor, 7863 Ghoy (BE); RASOLOFONIRINA, Richard, 601 Tuléar (MG); LEONET, Aline, 5555 Bièvre (BE)
(74) Mandataire: Bounaga, Sakina
(86) Numéro de dépôt international: PCT/EP2007/056665
(87) Numéro de publication internationale: WO 2008/003691

(56) Documents cités:
- KOMABA S ET AL: "Identification of myosin II kinase from sea urchin eggs as protein kinase CK2" GENE: AN INTERNATIONAL JOURNAL ON GENES AND GENOMES, ELSEVIER, AMSTERDAM, NL, vol. 275, no. 1, 5 septembre 2001 (2001-09-05), pages 141-148, XP004307121 ISSN: 0378-1119
- KISHIMOTO T ET AL: "GENERALITY OF THE ACTION OF VARIOUS MATURATION PROMOTING FACTORS" EXPERIMENTAL CELL RESEARCH, vol. 137, no. 1, 1982, pages 121-126, XP002470248 ISSN: 0014-4827
- MARUYAMA Y K: "INDUCTION OF SEA-CUCUMBER OOCYTE MATURATION BY STARFISH RADIAL NERVE EXTRACTS" JOURNAL OF EXPERIMENTAL ZOOLOGY, vol. 238, no. 2, 1986, pages 241-248, XP009096236 ISSN: 0022-104X
- CHEN C-P ET AL: "COMPARISON OF LARVAL DEVELOPMENT AND GROWTH OF THE SEA CUCUMBER ACTINOPYGA-ECHINITES OVARY-INDUCED OVA AND DTT-INDUCED OVA" MARINE BIOLOGY (BERLIN), vol. 109, no. 3, 1991, pages 453-458, XP009096239 ISSN: 0025-3162
- MARUYAMA Y K: "ARTIFICIAL INDUCTION OF OOCYTE MATURATION AND DEVELOPMENT IN THE SEA-CUCUMBERS HOLOTHURIA-LEUCOSPILOTA AND HOLOTHURIA-PARDALIS" BIOLOGICAL BULLETIN (WOODS HOLE), vol. 158, no. 3, 1980, pages 339-348, XP009096222 ISSN: 0006-3185
- VORONINA EKATERINA ET AL: "Cyclin B synthesis is required for sea urchin oocyte maturation." DEVELOPMENTAL BIOLOGY, vol. 256, no. 2, 15 avril 2003 (2003-04-15), pages 258-275, XP009096233 ISSN: 0012-1606
- KARASEVA E M ET AL: "Effect of compounds elevating cyclic nucleotide levels on dithiothreitol-induced oocyte maturation in the holothurian Stichopus japonicus" COMPARATIVE BIOCHEMISTRY AND PHYSIOLOGY C PHARMACOLOGY TOXICOLOGY AND ENDOCRINOLOGY, vol. 111, no. 3, 1995, pages 441-444, XP009096232 ISSN: 0742-8413
- SMILEY S: "A review of echinoderm oogenesis." JOURNAL OF ELECTRON MICROSCOPY TECHNIQUE OCT 1990, vol. 16, no. 2, octobre 1990 (1990-10), pages 93-114, XP009096251 ISSN: 0741-0581
- MORGAN ANDREW DAVID: "Aspects of the reproductive cycle of the sea cucumber Holothuria scabra (Echinodermata: Holothuroidea)" BULLETIN OF MARINE SCIENCE, vol. 66, no. 1, janvier 2000 (2000-01), pages 47-57, XP009096241 ISSN: 0007-4977

## Description

### Domaine de l'invention

La présente invention concerne la culture d'échinodermes et en particuliers de concombres de mer ou holothuries (holothuriculture). En particulier la présente invention concerne un procédé de maturation ovocytaire d'échinodermes et en particulier d'holothuries.

### Etat de la technique

Une des étapes cruciales en holothuriculture est l'obtention d'oeufs fécondés en grande quantité. Actuellement, la méthode utilisée consiste à soumettre les holothuries à des chocs thermiques. En pratique, les géniteurs sont transférés de manière répétée d'un aquarium à température ambiante vers un autre plus chaud de quelques degrés Celsius. Lorsque cette manipulation est effectuée pendant la période de reproduction, certains individus peuvent émettre leurs gamètes dans les heures qui suivent l'application des chocs thermiques. Toutefois la méthode n'est guère fiable et les résultats obtenus sont très aléatoires ; ils varient fortement non seulement en fonction du protocole mis en oeuvre, mais aussi selon l'espèce utilisée, voire même le géniteur manipulé.

Par ailleurs, il n'est actuellement pas possible d'effectuer des fécondations *in vitro* à partir des ovocytes prélevés dans des ovaires obtenus par dissection de femelles mûres. Chez les holothuries en effet, l'ovogenèse est bloquée au stade prophase de la première division de méiose. La levée de ce blocage, qui permet la poursuite de l'ovogénèse et donc la maturation ovocytaire qui, elle-même, permettra la fécondation, s'effectue naturellement juste avant la ponte de l'animal. En conséquence, chez les holothuries, le processus de maturation des ovocytes (processus qui implique, successivement, la migration du noyau vers la périphérie de la cellule, la disparition de la membrane nucléaire et la formation des deux globules polaires) a lieu dans le milieu extérieur. Mettre au point une méthode induisant efficacement la maturation ovocytaire d'holothuries exploitées commercialement serait d'un grand intérêt économique : cela permettrait l'obtention immédiate de centaines de milliers d'oeufs fécondables à partir d'un seul couple de géniteur (un ovaire de femelle mûre peut contenir plusieurs centaines de milliers d'ovocytes en attente de maturation). Dans ce but, des molécules connues pour induire la maturation ovocytaire (Maturation Inducing Substances ou MIS) chez certains invertébrés marins ont été essayées sur diverses espèces d'holothuries : les substances testées étaient la 1-méthyladenine (1 MéA), le dithiothréitol (DTT), le 2,3dimercapto-propanol (DMP ou BAL) et la L-cystéine (L-Cys). Toutes ces molécules se sont avérées non utilisables en holothuriculture car soit, elles n'induisent que de très faible taux de maturation des ovocytes, soit, lorsque l'induction se réalise, les ovocytes sont tantôt non fécondables, tantôt le sont mais donnent naissance à des larves anormales.

La présente invention a donc pour objet de remédier aux inconvénients cités ci-dessus. En particulier la présente invention à pour objet de fournir un procédé pour enclencher la maturation d'ovocytes chez les holothuries, en vue de l'obtention de larves viables en quantité illimitée.

### Bref résumé de l'invention

La demanderesse a trouvé qu'il était possible d'induire la maturation d'ovocytes d'échinodermes tels que les holothuries à l'aide d'un extrait d'échinides.

La présente invention concerne l'utilisation d'un extrait d'échinides pour induire la maturation d'ovocytes, de préférence l'extrait d'échinides est obtenu par un procédé comprenant l'extraction d'une fraction protéique à partir d'au moins un échinidé. De préférence, ladite extraction comprend l'isolation des gonades d'échinides.

La présente invention concerne aussi l'utilisation dudit extrait pour induire la maturation d'ovocytes d'invertébrés marins.

La présente invention concerne aussi un procédé pour la maturation d'ovocytes d'échinodermes, caractérisé en ce qu'un ou plusieurs ovocytes d'échinodermes sont mis en contact avec une quantité efficace d'un extrait d'échinides de sorte à obtenir des ovocytes matures.

La présente invention concerne donc aussi un procédé pour la maturation d'ovocytes d'holothuries, caractérisé en ce qu'un ou plusieurs ovocytes d'holothuries sont mis en contact avec une quantité efficace d'un extrait d'échinides de sorte à obtenir des ovocytes matures.

D'autres aspects, particularités et avantages de la présente invention apparaîtront à la lecture de la description qui va suivre et des exemples qui l'illustrent.

### Brève description de la figure

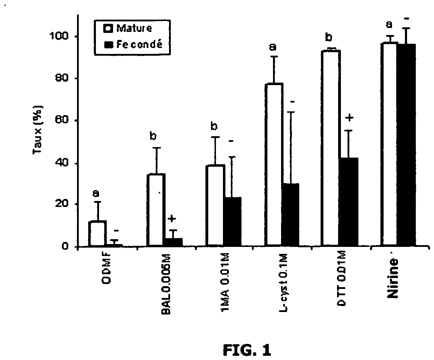

La Figure 1 représente un graphe montrant le taux de maturation et de fécondation des ovocytes de *Holothuria scabra* (avec écart type) après incubation en présence de différentes MIS et en présence de l'extrait d'échinoderme (dénommé ci-après *'la nirine*'). Deux signes consécutifs différents (+&- ou -8+ ou a&b ou b&a) indiquent des valeurs significativement différentes (test de Kruskal-Wallis ; seuil de signification = 0.05). Abréviations : eau de mer filtrée (ODM), 1-méthyladenine (1 MéA), le dimercapto-propanol (DMP ou BAL), la L-cystéine, le dithiothreitol (DTT).

### Description détaillée de l'invention

Les inventeurs ont découvert un inducteur efficace de maturation ovocytaire.

Le terme « maturation » comprend l'ensemble des étapes physiologiques et cytologiques rendant l'ovocyte apte à être fécondé. Le terme maturation ovocytaire se réfère parfois également à l'ensemble du processus de développement des gamètes femelles, devenant ainsi synonyme d'oogenèse.

La présente invention concerne aussi un procédé de maturation ovocytaire utilisant un extrait d'échinides obtenus par un procédé comprenant,
(a) l'isolation d'au moins une partie d'échinide,
(b) la centrifugation de l'isolat obtenu à l'étape (a) et
(c) la récupération du culot de centrifugation dudit isolat afin d'obtenir l'extrait d'échinide.

De préférence, ledit échinide est un Regularia (ou oursin régulier). Les Regularia se caractérisent par une symétrie radiaire (pentaradiaire) marquée, un test de forme plus ou moins globuleuse, une bouche et un anus diamétralement opposés. La bouche est au centre de la face orale et traverse une membrane péristoméale. L'anus est au centre de la face aborale et traverse une membrane périproctale inscrite dans un cercle apical encore appelé appareil apical.

Selon un mode de réalisation préféré, ledit échinide est sélectionné dans le groupe comprenant les Echinoidea, en particulier la sous-classe des Euechinoidea, en particulier le super-ordre des Echinacea, en particulier les ordres des Echinoida, des Physomatoida et des Temnopleurida, et parmi eux toutes les espèces appartenant aux genres *Anthocidaris, Arbacia, Colobocentrotus, Echinometra, Echinostrephus, Echinus, Evechinus, Heliocidaris, Heterocentrotus, Loxechinus, Lytechinus, Mespilia, Paracentrotus, Parasalenia, Parechinus, Pseudoboletia, Pseudocentrotus, Psammechinus, Salmacis, Sphaerechinus, Sterechinus, Stomechinus, Temnopleurus, Tetrapygus, Toxopneustes, Tripneustes, Stomopneustes, et Strongylocentrotus.*

Selon un mode de réalisation particulier, ledit procédé comprend à l'étape (a) l'isolation d'organes d'échinide de préférence, ledit procédé comprend l'isolation des gonades d'échinides, de préférence des gonades femelles, plus préférentiellement des ovocytes d'échinides. Par exemple, ladite étape (a) peut consister en l'isolation des pontes d'échinides.

Selon un mode de réalisation particulier, le culot de centrifugation de l'étape (c) peut être soit utilisé tel quel pour induire la maturation ovocytaire, ou peut être lyophilisé. Le lyophilisat peut être ensuite réduit en poudre.

Selon un mode de réalisation particulier, ledit extrait d'échinide obtenu à l'étape (c) peut être un extrait protéique.

Selon un mode de réalisation particulier, ledit extrait lyophilisé peut être fractionné par chromatographie. De préférence ledit extrait d'échinide obtenu par le procédé selon l'invention est un extrait protéique comprenant au moins une protéine d'au moins 12000 Da.

L'extrait d'échinides permet d'induire la maturation d'ovocytes d'échinodermes tels que les holothuries.

La présente invention concerne donc aussi l'utilisation d'un extrait d'échinides pour induire la maturation d'ovocytes d'échinodermes, tels que les échinodermes astérides, les échinodermes échinides, et les échinodermes holothuries. De préférence, la présente invention concerne l'utilisation d'un extrait d'échinoderme pour la maturation ovocytaire d'holothuries.

De préférence les holothuries sont des holothuries aspidochirotes, par exemple, celles-ci peuvent être sélectionnées parmi toutes les espèces appartenant aux genres *Actinopyga, Bohadschia, Holothuria, Isostichopus, Labidodemas, Thelenota, Pearsonothuria, Stichopus.*

La présente invention concerne aussi un procédé pour la maturation d'ovocytes d'échinodermes, caractérisé en ce qu'un ou plusieurs ovocytes d'échinodermes sont mis en contact avec une quantité efficace d'un extrait d'échinides de sorte à obtenir des ovocytes matures.

Selon un mode de réalisation particulier, ledit procédé comprend :
- le prélèvement des ovocytes sur des échinodermes,
- le traitement des ovocytes prélevés avec une quantité efficace dudit extrait de sorte à induire la maturation desdits ovocytes. De préférence l'extrait est un extrait d'échinidé, de préférence protéique.

Selon un mode de réalisation particulier, ledit extrait protéique est utilisé dans des concentrations allant de 10 % à 0.01‰, par exemple de 5% à 0.05 ‰, par exemple de 5% à 0.1 ‰, par exemple de 5 % à 0.5‰, par exemple à 40‰, 30‰, 20‰, 15‰, 10‰, 9‰, 8‰, 7‰, 6‰, 5‰, 4‰, 3‰, 2‰, 1‰, 0.8‰.

Obtenu à partir d'extraits d'échinides, cet inducteur, dénommé ci-après nirine, permet la maturation de plus de 90% des ovocytes au stade vitellogéniques contenu dans les ovaires d'échinodermes et en particulier d'holothuries, et ce quel que soit le moment de l'année (en d'autres termes l'emploi de la nirine permet l'obtention d'ovocytes matures -et de surcroît fécondables-pendant et en dehors de la période de reproduction naturelle de l'espèce). La nirine est active sur les ovaires des espèces d'holothuries Aspidochirotes, à savoir plusieurs centaines d'espèces parmi lesquelles toutes celles qui sont exploitées pour l'alimentation humaine. La nirine est de nature protéique ; à 4°C et sous forme lyophilisée elle garde son activité biologique pendant plus d'un an. Selon un mode de réalisation particulier, l'extrait d'échinide est un extrait aqueux. Ledit extrait peut être obtenu par broyage d'échinides, puis centrifugation et fractionnement du culot de centrifugation.

Selon un mode de réalisation particulier de l'invention, le procédé comprend après traitement (mise en contact) des ovocytes avec une quantité efficace dudit extrait, la maturation desdits ovocytes est induite. Le terme "quantité efficace" doit être compris comme un dosage d'extrait d'échinides adéquat aux résultats souhaités. Selon un mode de réalisation préféré, les ovocytes sont prélevés sur des holothuries Aspidochirotes utilisées en alimentation humaine.

La présente invention concerne aussi l'utilisation d'un extrait d'échinides pour la maturation d'ovocytes d'holothuries.

Le procédé de maturation et l'utilisation d'extrait d'échinides selon la présente invention sont d'un grand intérêt économique et permettent donc la mise au point d'une méthode induisant efficacement la maturation ovocytaire d'holothuries exploitées commercialement. La présente invention permet donc l'obtention immédiate de centaines de milliers d'oeufs fécondés par exemple à partir d'un seul couple de géniteur. L'invention permet de plus l'obtention de larves viables en quantité illimitée.

### Exemples

Les inventeurs ont découvert un inducteur de maturation, nommé ici « nirine », et ont mis au point une technique permettant la fécondation de plus de 90% des ovocytes matures prélevés par dissection sur des holothuries.

### Exemple 1 : Efficacité de la nirine comparée à d'autres molécules

Les molécules inductrices de maturations sont la 1-methyladénine (1Méa), du dithiothréitol (DTT), le dimercapto-propanol (BAL), et la L-cystéine (L-cyst). Chacune de ces substances ont été testées à différentes concentrations sur les ovocytes d'*Holothuria scabra.*

La figure 1 compare les taux de maturation (nombre d'ovocytes maturés/nombre d'ovocytes observés) et de fécondation (nombre d'oeufs fécondés/nombre d'oeufs observés) des ovocytes de *Holothuria scabra* placés dans de l'eau de mer (ODM) avec ceux incubés avec soit de la 1 MéA, du DMP (BAL), de la L-cystéine, du DTT ou de la nirine. Chaque molécule utilisée présente une dose optimale à laquelle un taux de maturation maximal a été observé. Le DMP et la 1MéA induisent moins de 40% de maturation. La L-cystéine et le DTT induisent plus de 70% de maturation mais moins de 50% des ovocytes traités avec une de ces molécules sont fécondables. Dans tous les cas, les oeufs fécondés donnent naissance à des larves anormales non viables au delà de 5 jours. D'autre part, l'incubation des ovocytes avec la nirine induit la maturation et permet la fécondation de plus de 90% d'entre eux ; les embryons obtenus sont viables et donnent des larves puis des juvéniles dont le développement se déroule en toute normalité.

### Exemple 2 : Effet de la nirine sur la maturation ovocytaire d'holothuries

Les ovocytes des holothuries ont été prélevés par dissections. Les gonades ont été récupérées à la main et rincées deux fois à l'eau de mer filtrée. Les tubules gonadiques ont ensuite été coupés en morceaux de 0.5 cm de longueur, ce qui a facilité la libération des gamètes. Les ovocytes ont alors été séparés des débris des tubules gonadiques par l'utilisation de tamis (vide de maille de 500gm de diamètre) et placés dans des récipients remplis d'eau de mer filtrée, à température ambiante. Seuls les ovocytes les plus gros ont été utilisés au cours du test.

Après deux heures d'incubation dans une des solutions décrites ci-dessous, la maturation ovocytaire a été vérifiée sous microscope en comptant à partir des cent premiers ovocytes observés sous microscope, le nombre d'ovocytes sans vésicule germinative et ayant expulsé les globules polaires. Le contrôle négatif consistait à incuber des ovocytes d'holothurie dans 10ml d'eau de mer filtrée. Le contrôle positif consistait à incuber les ovocytes dans une solution de nirine à une concentration déterminée (concentration en ovocytes 40/ml).

L'action de la nirine a été testée sur les ovocytes de différentes espèces d'holothuries : *Holothuria scabra, Holothuria leucospilota, Holothuria edulis, Holothuria fuscogilva, Holothuria maculosa, Bohadschia subrubra, Thetenota ananas,* et *Ho*/*othuria tubulosa.* Chez toutes les espèces citées une moyenne de 70% des ovocytes ont maturé en présence de nirine 2‰, contre seulement 17% pour des ovocytes placés en eau de mer filtrée. Ces espèces appartiennent à l'ordre des aspidochirotes.

D'autres espèces d'aspidochirotes ont été testées : *Holothuria cinerascens, Pearsonothuria graeffei, Actinopyga echinites.* Le pourcentage de maturation des ovocytes de ces trois espèces d'aspidochirotes, incubés dans une solution de nirine 2‰, est en moyenne de 88% contre 25% pour les ovocytes places dans de l'eau de mer.

### Exemple 3 : Extraction de l'extrait protéique inducteur de maturation

L'extrait protéique selon un mode de réalisation préféré, consiste en un lyophilisat du culot de centrifugation des pontes de l'oursin *Tripneustes gratilla.* La propriété d'induire la maturation des ovocytes d'holothurie n'est pas propre aux pontes de *Tripneustes gratilla* car les pontes des oursins réguliers *Stomopneustes variolaris, Echinometra mathaei* et *Paracentrotus lividus* donnaient de très bon résultats (une moyenne de 92% de maturation ovocytaire contre 19% dans le témoin).

Pour obtenir l'extrait protéique, les pontes de ces oursins ont tout d'abord été provoquées. Celles-ci ont été induites par stress mécanique (les animaux ont été agités manuellement durant quelques minutes ce qui provoquait spontanément l'émission des gamètes lorsque l'animai était à maturité). Les ovocytes ainsi pondus ont été centrifugés 10 minutes à 5000 rpm. Le produit inducteur de maturation a été préparé à partir des culots de pontes fraîches centrifugées à une vitesse de 5000 rpm (rotation par minute) pendant 2x10 min. Les culots des centrifugations ont été soit utilisés extemporanément, soit congelés, lyophilises (24h) réduits en poudre et conserves au frigo jusqu'au moment des expérimentations.

Cette poudre d'extrait protéique a été diluée dans de l'eau de mer à concentration adéquate (2‰) et la solution a été filtrée avant expérimentation.

L'utilisation de membrane à dialyse a permis de confirmer le poids moléculaire minimum de l'agent inducteur de maturation ovocytaire contenu dans l'extrait protéique. Pour cela, trois ml d'extrait protéique (2‰) ont été placés dans une membrane à dialyse (SPECTRUM) présentant une barrière d'exclusion (cut off) de 12000 Daltons. Cette membrane a été immergée sous agitation dans un bécher contenant 31 de tampon Tris. Les 31 de tampon ont été changés trois fois sur 24 heures. La nirine contenue dans la membrane a été récupérée et testée sur des ovocytes d'*Holothuria scabra.* Les tests de maturation ont montré que l'agent actif a gardé toute son efficacité après dialyse de 24h que ce soit contre de l'eau de mer ou le tampon. L'agent actif fait donc plus de 12000Da. L'action d'une protéinase K, enzyme digérant les protéines avec un large spectre d'action, a permis de démontrer que l'agent inducteur de maturation présent dans les fractions actives obtenues par chromatographie est de nature protéique. A cet effet, les fractions actives obtenues par chromatographies ont été soumises à l'action de la protéinase K (concentration de 0.01mg/ml) durant 3h a 40° à pH 7. L'effet de la protéinase K a été inhibé en chauffant cette solution à 100°C pendant 30 minutes. L'activité de cette solution, refroidie à température ambiante, a été testée sur des ovocytes d'*Holothuria scabra.*

## Revendications

1. Utilisation d'un extrait d'échinides pour induire la maturation d'ovocytes d'échinodermes et plus préférentiellement d'holothuries.

2. Utilisation selon la revendication 1, **caractérisée en ce que** lesdits échinides sont des oursins réguliers.

3. Utilisation selon les revendications 1 ou 2, **caractérisée en ce que** ledit extrait d'échinides est susceptible d'être obtenu par un procédé comprenant,
a) l'isolation d'au moins une partie d'échinides
b) la centrifugation de l'isolat obtenu à l'étape a) et
c) la récupération du culot de centrifugation dudit isolat afin d'obtenir l'extrait d'échinides.

4. Utilisation selon l'une quelconque des revendications 1 à 3, **caractérisée en ce que** ledit extrait d'échinides est un extrait protéique.

5. Utilisation selon la revendication 4, **caractérisée en ce que** l'extrait protéïque est susceptible d'être obtenu des pontes d'échinides.

6. Utilisation selon l'une quelconque des revendications 1 ou 5, **caractérisée en ce que** l'extrait d'échinides comprend au moins une protéine ayant un poids moléculaire supérieur à 12000 Dalton.

7. Procédé pour la maturation d'ovocytes d'échinodermes, **caractérisé en ce qu'**un ou plusieurs ovocytes d'échinodermes sont mis en contact avec une quantité efficace d'un extrait d'échinides de sorte à obtenir des ovocytes matures.

8. Procédé selon la revendication 7, **caractérisé en ce que** ledit extrait d'échinides est un extrait susceptible d'être obtenu selon le procédé comprenant :
a) l'isolation d'au moins une partie d'échinides
b) la centrifugation de l'isolat obtenu à l'étape a) et
c) la récupération du culot de centrifugation dudit isolat afin d'obtenir l'extrait d'échinides.

9. Procédé selon la revendication 7 ou 8, **caractérisé en ce qu'**il comprend :
le prélèvement des ovocytes d'échinodermes,
le traitement des ovocytes prélevés avec une quantité efficace dudit extrait de sorte à induire la maturation desdits ovocytes.

10. Procédé selon l'une quelconque des revendications 7 à 9, **caractérisé en ce que** l'extrait d'échinides est un extrait protéique.

11. Procédé selon l'une quelconque des revendications 7 à 10, **caractérisé en ce que** l'extrait d'échinides comprend au moins une protéine ayant un poids moléculaire supérieur à 12000 Dalton.

12. Procédé selon l'une quelconque des revendications 7 à 11, **caractérisé en ce que** l'extrait protéique est susceptible d'être obtenu des pontes d'échinides.

13. Procédé selon l'une quelconque des revendications 7 à 12 **caractérisé en ce que** ledit extrait protéique est utilisé dans des concentrations allant de 10 % à 0.01‰.

## Claims

1. Use of an extract from echinoids to induce the maturation of oocytes from echinoderms, more preferably from holothurians.

2. Use according to claim 1, **characterized in that** said echinoids are regular sea urchins.

3. Use according to claim 1 or 2, **characterized in that** said echinoid extract can be obtained by a method comprising:
a) isolating at least one part of the echinoid;
b) centrifugation of the isolate obtained in step a); and
c) recovery the centrifugation pellet from said isolate in order to obtain the echinoid extract.

4. Use according to any one of claims 1 to 3, **characterized in that** said echinoid extract is a proteinaceous extract.

5. Use according to claim 4, **characterized in that** the proteinaceous extract can be obtained from spawns of said echinoids.

6. Use according to any of claim 1 or 5, **characterized in that** the echinoid extract comprises at least one protein having a molecular weight higher than 12000 Dalton.

7. A process for the maturation of echinoderm oocytes, **characterized in that** one or more echinoderm oocytes are brought into contact with an effective quantity of an echinoid extract so as to obtain mature oocytes.

8. A process according to claim 7, **characterized in that** said echinoid extract is an extract which can be obtained in accordance with the method comprising:
a) isolating at least one part of the echinoid;
b) centrifugation of the isolate obtained in step a); and
c) recovery of the centrifugation pellet from said isolate in order to obtain the echinoid extract.

9. A process according to claim 7 or 8, **characterized in that** it comprises:
sampling oocytes from echinoderms;
treatment of the sampled oocytes with an effective quantity of said extract so as to induce maturation of said oocytes.

10. A process according to any one of claims 7 to 9, **characterized in that** the echinoid extract is a proteinaceous extract.

11. A process according to any one of claims 7 to 10, **characterized in that** the echinoid extract comprises at least one protein having a molecular weight higher than 12000 Dalton.

12. A process according to any one of claims 7 to 11, **characterized in that** the proteinaceous extract can be obtained from spawns of said echinoids.

13. A process according to any one of claims 7 to 12, **characterized in that** said proteinaceous extract is used in concentrations ranging from 10% to 0.01‰.

## Patentansprüche

1. Verwendung eines Echinidenextrakts, um die Oozytenreifung von Echinodermata und insbesondere Holothuriodia zu induzieren.

2. Verwendung nach Anspruch 1, **dadurch gekennzeichnet, dass** die Echiniden gewöhnliche Seeigel sind.

3. Verwendung nach den Ansprüchen 1 oder 2, **dadurch gekennzeichnet, dass** der Echinidenextrakt durch ein Verfahren erhalten werden kann, umfassend,
(a) Isolieren mindestens eines Echinidenteils,
(b) Zentrifugieren des in Schritt (a) erhaltenen Isolats, und
(c) Gewinnen des Zentrifugationspellets des Isolats, um den Echinidenextrakt zu erhalten.

4. Verwendung nach einem der Ansprüche 1 bis 3, **dadurch gekennzeichnet, dass** der Echinidenextrakt ein Proteinextrakt ist.

5. Verwendung nach Anspruch 4, **dadurch gekennzeichnet, dass** der Proteinextrakt aus Echinidenlaichen erhalten werden kann.

6. Verwendung nach einem der Ansprüche 1 oder 5, **dadurch gekennzeichnet, dass** der Echinidenextrakt mindestens ein Protein umfasst, das ein Molekulargewicht oberhalb von 12.000 Dalton aufweist.

7. Verfahren zur Oozytenreifung von Echinodermata, **dadurch gekennzeichnet, dass** ein oder mehrere Oozyten von Echinodermata mit einer wirksamen Menge eines Echinidenextrakts in Kontakt gebracht werden, um reife Oozyten zu erhalten.

8. Verfahren nach Anspruch 7, **dadurch gekennzeichnet, dass** der Echinidenextrakt ein Extrakt ist, der gemäß dem Verfahren erhalten werden kann, umfassend:
a) Isolieren mindestens eines Echinidenteils,
b) Zentrifugieren des in Schritt a) erhaltenen Isolats, und
c) Gewinnen des Zentrifugationspellets des Isolats, um den Echinidenextrakt zu erhalten.

9. Verfahren nach Anspruch 7 oder 8, **dadurch gekennzeichnet, dass** es umfasst:
das Entnehmen der Echinodermata-Oozyten,
das Behandeln der entnommenen Oozyten mit einer wirksamen Menge des Extrakts, um die Reifung der Oozyten zu induzieren.

10. Verfahren nach einem der Ansprüche 7 bis 9, **dadurch gekennzeichnet, dass** der Echinidenextrakt ein Proteinextrakt ist.

11. Verfahren nach einem der Ansprüche 7 bis 10, **dadurch gekennzeichnet, dass** der Echinidenextrakt mindestens ein Protein umfasst, das ein Molekulargewicht oberhalb von 12.000 Dalton aufweist.

12. Verfahren nach einem der Ansprüche 7 bis 11, **dadurch gekennzeichnet, dass** der Proteinextrakt aus Echinidenlaichen erhalten werden kann.

13. Verfahren nach einem der Ansprüche 7 bis 12, **dadurch gekennzeichnet, dass** der Proteinextrakt in Konzentrationen im Bereich von 10 % bis 0,01 ‰ verwendet wird.
